(19) Europäisches Patentamt
European Patent Office
Office européen des brevets

(11) EP 3 339 289 A1

(12) EUROPÄISCHE PATENTANMELDUNG

(43) Veröffentlichungstag:
**27.06.2018 Patentblatt 2018/26**

(21) Anmeldenummer: **16205829.1**

(22) Anmeldetag: **21.12.2016**

(51) Int Cl.:
***C07C 319/20*** *(2006.01)*
***C01C 3/02*** *(2006.01)*
***C07D 233/76*** *(2006.01)*
***C07C 323/60*** *(2006.01)*
***C07C 323/58*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Degussa GmbH**
**45128 Essen (DE)**

(72) Erfinder:
- **HIEROLD, Judith**
  **30161 Hannover (DE)**
- **SUDHOFF, Daniel**
  **63456 Hanau (DE)**
- **STEURENTHALER, Martin**
  **098654 Singapore (SG)**
- **HASSELBACH, Hans Joachim**
  **63571 Gelnhausen (DE)**
- **ROTH, Philipp**
  **63456 Hanau (DE)**
- **MERKER, Thorsten**
  **63456 Hanau (DE)**
- **HELD, Markus**
  **63694 Limeshain (DE)**
- **FISCHER, Daniel**
  **Midlothian, VA 23113 (US)**
- **KAISER, Christian**
  **63857 Waldaschaff (DE)**

# (54) VERFAHREN ZUR HERSTELLUNG VON METHIONIN

(57) Die vorliegende Erfindung betrifft ein Verfahren zur Herstellung von Methionin oder Methioninsalzen. Insbesondere beschreibt die Erfindung den Schritt der Herstellung von 2-Hydroxy-4-(methylthio)buttersäurenitril (MMP-CN) aus 3-Methylthiopropanal (MMP) und Cyanwasserstoff (HCN) in Gegenwart von Ammoniak durch das In-Kontakt-Bringen eines gasförmigen Gemisches enthaltend HCN und Ammoniak mit MMP.

EP 3 339 289 A1

**Beschreibung**

Die vorliegende Erfindung betrifft ein Verfahren zur Herstellung von Methionin oder dessen Salzen. Insbesondere beschreibt die Erfindung die Herstellung von 2-Hydroxy-4-(methylthio)buttersäurenitril (MMP-CN) aus 3-(Methylthio)propanal (= Methylmercaptopropionaldehyd, MMP) und ammoniakhaltigem Cyanwasserstoff (Blausäure, HCN).

2-Hydroxy-4-(methylthio)buttersäurenitril (MMP-CN) ist unter anderem ein Intermediat für die Darstellung von Methionin. Methionin ist eine essentielle Aminosäure, die u.a. als Ergänzung in Futtermitteln eingesetzt wird. Nähere Informationen sind in einer Vielzahl von Fachbüchern zu finden, zum Beispiel in Ullmann's Encyclopedia of Industrial Chemistry, Kapitel "Amino Acids" veröffentlicht online am 15.04.2007, DOI: 10.1002/14356007.a02_057.pub2.

In vielen gängigen Verfahren wird Methionin als Racemat, einem Gemisch aus dem D- und L-Enantiomer, auf einem mehrstufigen chemischen Weg, zum Beispiel über die sogenannte Hydantoinroute aus Acrolein, Methylmercaptan, Cyanwasserstoff und Ammoniumkarbonat hergestellt:

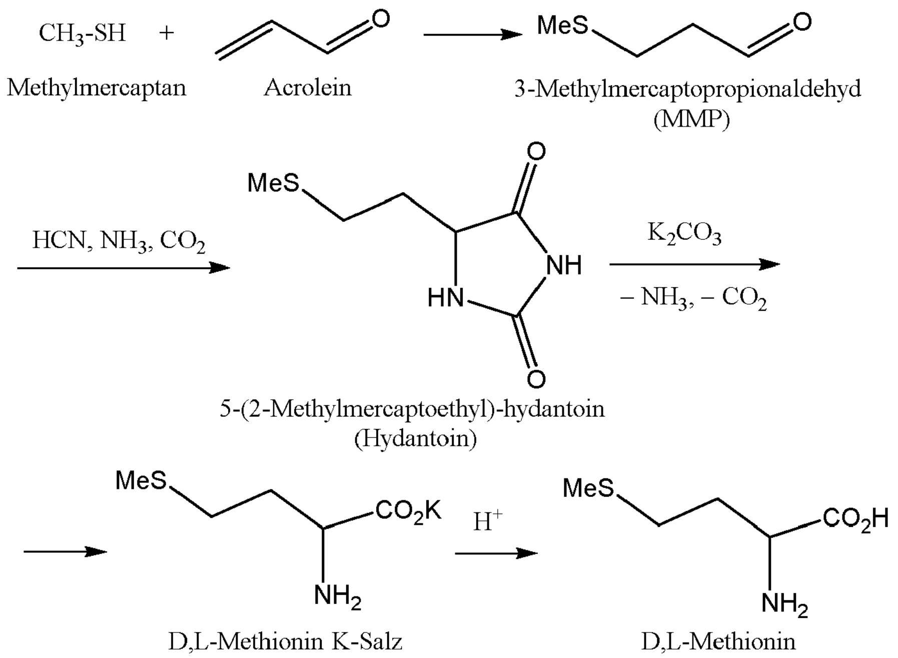


Die Durchführung des zweiten Schritts, der Synthese von 5-(2-Methylmercaptoethyl)-hydantoin (Hydantoin), kann dabei direkt aus MMP, HCN, Ammoniak und $CO_2$ oder alternativ zweistufig über die Bildung der Cyanhydrinvorstufe MMP-CN erfolgen:

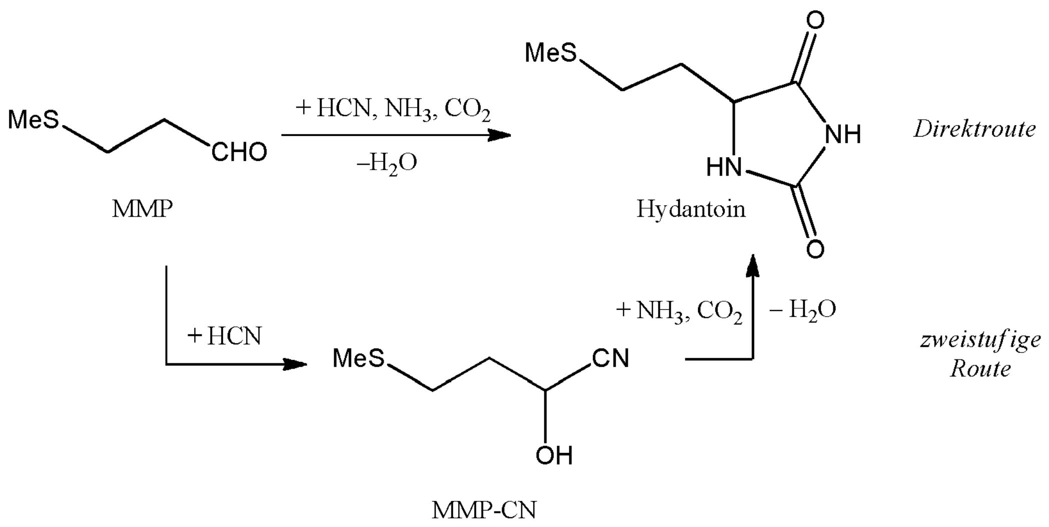


EP 0780370 A2 offenbart ein Verfahren zur Herstellung von Methionin, bei dem MMP, HCN, Ammoniak und

Kohlendioxid zu einer Methionin-Vorstufe, Hydantoin, reagieren (*Direktsynthese des Hydantoins*). Das molare Verhältnis von Ammoniak zu MMP kann dabei zwischen 1,2 und 6 liegen, das molare Verhältnis von Ammoniak zu Kohlendioxid beträgt 1,2 bis 4,0. Nach diesem Verfahren wird MMP in praktisch quantitativer Ausbeute zum Hydantoin umgesetzt. Das so hergestellte Hydantoin wird dann weiter unter Bildung eines Methioninsalzes gespalten. Das bei der Spaltung entstehende Ammoniak und Kohlendioxid kann im Prozess rezykliert werden.

In JP 2003104959 A und JP 2003104960 A ist ein ähnliches Verfahren zur Umsetzung von MMP mit HCN, $NH_3$ und $CO_2$ zum Hydantoin beschrieben. Dabei können die molaren Verhältnisse von Ammoniak zu MMP und $CO_2$ zu MMP Werte von jeweils 1,5 bis 2,5 annehmen.

In den früheren Anmeldungen GB 936664 A und GB 1108926 A ist ebenfalls ein Verfahren zur Umsetzung von MMP mit HCN, $NH_3$ und $CO_2$ zum Hydantoin durch die Reaktion von Aldehyden oder Ketonen mit einer äquimolaren oder überschüssigen Menge an $CO_2$ und einem Überschuss an Ammoniak in wässrigem Milieu beschrieben.

WO2012/113664 A1 und WO 2012/113665 A1 beschreiben ein Verfahren zur Herstellung von MMP-CN aus MMP und HCN, den ersten Schritt der oben genannten zweistufigen Route zum Hydantoin.

Eine Vielzahl an Verfahren zur Herstellung von Cyanwasserstoff ist in der Literatur beschrieben. Die in der industriellen Praxis am häufigsten verwendeten sind das sogenannte Andrussow-Verfahren (DE 102007034715A1 und Chemie-Ing.-Techn., 1953, Nr. 12, S 697-701) und das sogenannte BMA-Verfahren (Blausäure aus Methan und Ammoniak, DE1041476 und Chemie-Ing.-Techn., 1958, Nr. 5, S 305-310 [1]). Während beim reduzierenden BMA-Verfahren Methan und Ammoniak zu Blausäure umgesetzt werden, geht man beim oxidierenden Andrussow-Verfahren von Methan, Ammoniak und Sauerstoff aus. Beide Verfahren sind näher beschrieben in "Handbook of Heterogeneous Catalysis", Herausgeber: G. Ertl. et al, zweite Auflage, Vol. 5, Kapitel 12.3 "Hydrocyanic Acid (HCN) Production" 2592-2609 [2] und in Ullmann's Encyclopedia of Industrial Chemistry, "Cyano Compounds, Inorganic" veröffentlicht Online am 15,10.2011, DOI: 10.1002/14356007.a08_159.pub3 [3].

Sowohl das Andrussow- als auch das BMA-Verfahren verläuft unter nicht-vollständigem Umsatz bezogen auf Ammoniak. Der den Reaktor verlassende HCN-Rohgasstrom enthält daher in beiden Fällen Anteile an Ammoniak. In der Literatur finden sich für dieses gasförmige Produktgemisch direkt nach der HCN-Herstellung folgende typische Gehalte an HCN und Ammoniak (in Vol%):

Tabelle 1:

| Verfahren | HCN (Vol%) | $NH_3$ (Vol%) | $NH_3$/HCN (mol/mol) | Referenz |
|---|---|---|---|---|
| Andrussow | 7,0 | 1,7 | 0,24 | [2] |
| Andrussow | 7,6 | 2,3 | 0,30 | [3] |
| Andrussow | 6,6 | 2,6 | 0,39 | [1] |
| BMA | 22,9 | 2,5 | 0,11 | [3] |

Das in Tabelle 1 dargestellte molare Verhältnis $NH_3$/HCN (mol/mol) ergibt sich aufgrund des Avogadro'schen Gesetzes aus den entsprechenden Konzentrationen an Ammoniak und Blausäure in Vol%:

$$NH_3/HCN\ (mol/mol) = [NH_3\ (Vol\%)\ im\ Produktgemisch]/[HCN\ (Vol\%)\ im\ Produktgemisch]$$

CN103408498A offenbart zudem den folgenden Konzentrationsbereich für $NH_3$ und HCN im HCN-Rohgasstrom nach dem Andrussow-Verfahren: 1,6±2 Gew% $NH_3$ und 8,8±2 Gew% HCN. Das entsprechende molare Verhältnis $NH_3$/HCN lässt sich daraus zu maximal 0,84 berechnen.

Das gasförmige Produktgemisch direkt nach der HCN-Synthese nach dem Andrussow-Verfahren enthält zusätzlich zu HCN und Ammoniak vor allem Wasser, aber auch Methan, Stickstoff, Wasserstoff, CO und $CO_2$ und andere Bestandteile. Eine typische Zusammensetzung für einen nach dem Andrussow-Verfahren hergestellten HCN-Rohgasstrom ist: HCN: 7,6 Vol%, $NH_3$: 2,3 Vol% und $H_2O$: 23,1 Vol% [3]. Typische Ausbeuten bei der Blausäure-Bildung bezogen auf Ammoniak betragen etwa 63%.

Im BMA-Verfahren enthält der HCN-Rohgasstrom zusätzlich zu HCN und Ammoniak vor allem Wasserstoff, aber auch Methan, Stickstoff und weitere Bestandteile. Eine typische Zusammensetzung für einen nach dem BMA-Verfahren hergestellten HCN-Rohgasstrom ist: HCN: 22,9 Vol%, $NH_3$: 2,5 Vol% und $H_2$: 71,8 Vol% [3]. Typische Blausäure-Ausbeuten bezogen auf Ammoniak betragen etwa 83%.

Das HCN-Rohgasgemisch liegt in beiden Verfahren direkt nach der Herstellung typischerweise bei Temperaturen von über 1000 °C vor und wird sofort abgekühlt. Der im HCN-Rohgasstrom enthaltene Ammoniak wird in allen gängigen Verfahren unverzüglich entfernt, zum Beispiel durch eine Waschung mit verdünnter Schwefelsäure. Dies ist

besonders zur Vermeidung von autokatalytischer exothermer Blausäure-Polymerisation nötig, welche insbesondere bei einem erhöhten pH und an den Phasenübergängen wie zum Beispiel bei der Verflüssigung von HCN stattfindet.

Wird reine Blausäure benötigt, so wird nach der Entfernung des Ammoniaks das Rohprodukt für gewöhnlich zur Abtrennung der Inertgase in kaltem Wasser absorbiert und später destillativ aufgereinigt. Die aufgereinigte Blausäure kann dann entweder gasförmig oder, nach Säurestabilisierung und Kondensation, flüssig in weiteren Prozessschritten, zum Beispiel im Verfahren zur Herstellung von Methionin, eingesetzt werden.

Laut Chemie-Ing.-Techn., 1953, Nr. 12, S 697-701 werden beim Andrussow-Verfahren etwa 60% des eingesetzten Ammoniaks zu Blausäure umgesetzt, 10% gehen über das Abgas verloren, 30% werden in der Säurewäsche mit Schwefelsäure als Ammoniumsulfat gebunden.

Nach dem BMA-Verfahren werden HCN-Ausbeuten von über 80% bezogen auf Ammoniak erreicht, wobei dem Prozess immer noch mindestens 10% des Ammoniaks in einer Säurewäsche entzogen werden müssen (Chemie-Ing.-Techn., 1958, Nr. 5, S 305-310).

Die durch Säurewaschung mit Schwefelsäure resultierenden Probleme des Ammoniumsulfat-Zwangsanfalls und des Verlustes an eingesetztem $NH_3$ sind in der Fachwelt seit langem bekannt, und es wurden mehrere Alternativlösungen zu dieser Problematik vorgeschlagen.

US 2590146 A offenbart die Absorption des HCN-Rohgasgemisches, hergestellt nach dem Andrussow-Verfahren, in einer wässrigen Lösung eines Borsäure-Pentaerythritol-Komplexes, wobei Ammoniak reversibel chemisch gebunden wird. Die nicht-gebundene Blausäure wird nachfolgend destillativ abgetrennt, gefolgt durch Zersetzung des Ammoniak-Borsäure-Pentaerythritol-Komplexes und Abtrennung des freigesetzten Ammoniaks. Dieses mehrstufige Verfahren ermöglicht somit die Abtrennung von HCN und Ammoniak aus dem HCN-Rohgasgemisch.

Das Prinzip der reversiblen Bindung von Ammoniak aus dem HCN-Rohgasstrom wird auch gemäß US 2899274 A verfolgt. Darin wird der Einsatz von wässriger Lösung von gesättigten Fettsäuren und deren Ammoniumsalzen als Absorptionsmedium für Ammoniak beschrieben.

US 3112177 A offenbart die Nutzung von $CO_2$ in Wasser, um Ammoniak reversibel zu binden; US 2797148 A und US 3914386 A schlagen Ammoniumhydrogenphosphatlösung für den gleichen Zweck vor.

Alle diese Methoden sind technisch sehr aufwendig; außerdem ist die reversible Absorption und Desorption von Ammoniak nie vollständig und muss gegebenenfalls mit einer nachgeschalteten Schwefelsäurewäsche ergänzt werden. Daher bleibt die Durchführung der HCN-Synthese mit nachfolgender Säurewaschung unter Ammoniumsulfatbildung bisher die gängigste industrielle Praxis.
Allen bekannten Methoden gemein ist die schnelle Abtrennung von Ammoniak aus dem HCN-Rohgasstrom mittels einer sauren Gaswäsche.

Vorteilhaft wäre es, auf die Abtrennung von Ammoniak und anderen Bestandteilen des HCN-Rohgasgemischs ganz oder teilweise verzichten zu können. Dies ist aber nach dem derzeitigen Stand der Technik nicht möglich. Neben der oben genannten Tendenz von flüssiger Blausäure zur Polymerisation in Gegenwart von Ammoniak bringt Ammoniak auch weitere Probleme in den nachfolgenden Prozessschritten der Methioninsynthese mit sich. So führt die Durchführung der Cyanhydrinsynthese, der Reaktion von MMP zu MMP-CN, in Gegenwart einer Base laut US 5,756,803 zwar zur Beschleunigung dieser Reaktion, fördert aber gleichzeitig eine schnellere Zersetzung des gebildeten Cyanhydrins und des eingesetzten Aldehyds, was in einer intensiven Verfärbung des Reaktionsgemisches resultiert. Um dieses Problem zu umgehen, wird daher in US 5756803 A vorgeschlagen, kein Amin bei der Cyanhydrinbildung einzusetzen.

US 2745745 erwähnt die Herstellung eines Cyanhydrins, beispielsweise MMP-CN, durch eine katalysierte Reaktion von MMP mit wasserfreier, flüssiger Blausäure. Als Katalysator können dabei Pyridin oder andere basischen Amine fungieren.

WO2012/113664 A1 und WO 2012/113665 A1 beschreiben ein weiteres katalytisches Verfahren zur Herstellung eines lagerstabilen Cyanhydrins MMP-CN aus MMP und gasförmiger Blausäure. Als Katalysator wird dabei ein Trialkylamin eingesetzt. Durch die Auswahl geeigneter Zusatzstoffe und Lagerbedingungen wurde MMP-CN mit hoher Lagerstabilität erhalten. Es wird in der Beschreibung darauf hingewiesen, dass nach der Entfernung des Ammoniaks aus dem HCN-Rohgasgemisch durch Säurewäsche von weiteren Aufreinigungsschritten vor der MMP-CN Synthese abgesehen werden kann.

CN 103408498 A und CN 103420883 A offenbaren ebenfalls eine Synthese von MMP-CN aus MMP und nicht destillierter HCN. Blausäure wird dabei durch das Andrussow-Verfahren gewonnen und vor der Cyanhydrinsynthese durch eine Absorption mit Schwefelsäure vom Ammoniak befreit.

In JP2002105048 ist eine durch Ammoniak katalysierte Synthese von MMP-CN aus MMP und HCN offenbart. Eine reine, ammoniakfreie Blausäure wird als wässrige Lösung mit MMP vermischt.

Zu dieser Mischung wird dann ein Amin, zum Beispiel Ammoniak, in katalytischen Mengen gegeben. Die Menge an eingesetztem Katalysator kann nach dieser Anmeldung 0,001 bis 0,05 mol auf 1 mol MMP betragen, das molare Verhältnis von HCN zu MMP nimmt die Werte von 1,0 bis 1,1 an, daraus errechnet sich das mögliche Molarverhältnis von Ammoniak zu HCN von 0,0009 bis 0,05.

Diese Auflistung zeigt, dass in jedem bekannten Verfahren die Blausäure nach der Herstellung direkt durch

eine saure Wäsche vom Ammoniak befreit wird. In keiner der vorliegenden Veröffentlichungen wird eine Synthese von MMP-Cyanhydrin aus MMP und gasförmiger Blausäure, welche zuvor nicht von Ammoniak befreit wurde, oder die Umsetzung eines so hergestellten MMP-Cyanhydrins zu Methionin oder anderen Folgeprodukten, offenbart.

Die Aufgabe der vorliegenden Erfindung ist es, ein wirtschaftliches Verfahren zur Herstellung von MMP-Cyanhydrin und dessen Folgeprodukten, insbesondere Methionin, aus nicht-aufgereinigter, gasförmiger Blausäure in hoher Gesamtausbeute und Reinheit bereitzustellen.

Das vorrangige Ziel ist dabei, den Aufwand der Abtrennung von Ammoniak aus dem HCN-Rohgasstrom zu minimieren und durch Eliminierung der Säurewäsche das Verfahren zu vereinfachen. Diese Prozessintensivierung ermöglicht eine Reduzierung der Gesamtzahl an Verfahrensschritten der Methioninsynthese. Zusätzlich wird durch diese vereinfachte HCN-Synthese die Bildung von Ammoniumsalzen vermieden und somit die Menge an Nebenprodukten der Methioninsynthese reduziert. Weiterhin ist der spezifische Bedarf an Ammoniak für die Methionin-Herstellung reduziert, da der überschüssige Ammoniak im HCN-Rohgasgemisch nicht entfernt sondern in die nachfolgenden Prozessschritte geleitet wird.

Die gestellten technischen Aufgaben werden durch ein Verfahren zur Herstellung von 2-Hydroxy-4-(methylthio)buttersäurenitril (MMP-CN), umfassend einen Schritt B, in dem ein Gasgemisch enthaltend Cyanwasserstoff (HCN) und Ammoniak mit 3-Methylmercaptopropionaldehyd (MMP) in Kontakt gebracht wird und dadurch ein Produktgemisch umfassend 2-Hydroxy-4-(methylthio)buttersäurenitril (MMP-CN) erhalten wird, gelöst.

Ein weiterer Gegenstand der Erfindung ist ein Verfahren zur Herstellung von Methionin oder einem Salz des Methionins, umfassend einen Schritt B, in dem ein Gasgemisch enthaltend Cyanwasserstoff (HCN) und Ammoniak mit 3-Methylmercaptopropionaldehyd (MMP) in Kontakt gebracht wird und dadurch ein Produktgemisch umfassend 2-Hydroxy-4-(methylthio)buttersäurenitril (MMP-CN) erhalten wird.
MMP-CN kann dann weiter umgesetzt werden zu Methionin(salz) beispielsweise gemäß einem der oben genannten Verfahren aus dem Stand der Technik.

Nachfolgend in Tabelle 2 werden die in der Beschreibung verwendeten chemischen Namen, deren Abkürzungen und deren entsprechende systematische Namen nach IUPAC sowie die CAS-Nummern aufgelistet:

Tabelle 2:

| Name | Abkürzung | IUPAC Name | CAS-Nr. |
|---|---|---|---|
| 3-Methylmercaptopropionaldehyd | MMP | 3-(Methylsulfanyl)propanal | 3268-49-3 |
| 2-Hydroxy-4-(Methylthio)-buttersäurenitril | MMP-CN | 2-Hydroxy-4-Methylsulfanylbutannitril | 17773-41-0 |
| 2-Amino-4-(Methylthio)-buttersäurenitril | MMP-AN | 2-Amino-4-(methylsulfanyl)butannitril | 3198-47-8 |
| 5-(2-Methylmercaptoethyl)-hydantoin | Hydantoin | 5-(2-Methylsulfanylethyl)-imidazolidin-2,4-dion | 13253-44-6 |
| | Iminodinitril | 2,2'-Bis-(2-(methylmercaptoethyl)-iminodiacetonitril | 1807317-18-5 |

Im Folgenden wird die Erfindung anhand von Fig. 1, die eine spezielle Ausführungsform der vorliegenden Erfindung darstellt, die besonders zur Herstellung eines Methioninsalzes geeignet ist, näher erläutert. Diese stark vereinfachte Zeichnung soll einen Gesamtüberblick der erfindungsgemäßen Verfahrensschritte geben. Im Folgenden (Tabelle 3) werden die Prozessschritte A bis F, die entsprechenden Ströme (1) bis (15) und die dazugehörenden Gemische näher beschrieben.

Tabelle 3: Bezugszeichen und Erklärungen zu Figur 1

| | |
|---|---|
| A | HCN-Synthese (nach Andrussow- oder BMA-Verfahren) |
| B | MMP-CN-Synthese aus MMP und HCN-Rohgasgemisch aus A |
| C | optionale MMP-CN-Zwischenlagerung |
| D | Hydantoin-Synthese |
| E | Hydantoin-Spaltung |
| F | optionale Aufreinigung von Ammoniak |
| (1) | Eduktgemisch enthaltend $CH_4$ und $NH_3$ zu HCN-Reaktor A |
| (2) | optional $N_2$ und/oder $O_2$ zu HCN-Reaktor A |
| (3) | Ammoniakhaltiges HCN-Rohgasgemisch zu MMP-CN-Synthese B |
| (4) | MMP zu MMP-CN-Synthese B |
| (5) | MMP-CN-Gemisch zu MMP-CN-Zwischenlagerung C |
| (6) | Abgas aus MMP-CN-Synthese B |
| (7) | MMP-CN-Gemisch zu Hydantoin-Synthese D |
| (8) | Gemisch enthaltend Hydantoin zu Hydantoin-Spaltung E |
| (9) | Gemisch enthaltend $NH_3$, $CO_2$ und $H_2O$ aus Hydantoin-Spaltung E |
| (10) | Gemisch enthaltend $NH_3$, $CO_2$ und $H_2O$ zu Hydantoin-Synthese D |
| (11) | Gemisch enthaltend $NH_3$, $CO_2$ und $H_2O$ zu weiterer Verwertung |
| (12) | Gemisch enthaltend $NH_3$ zu HCN-Synthese A |
| (13) | Gemisch enthaltend $NH_3$, $CO_2$ und $H_2O$ zur weiteren Verwertung und/oder Entsorgung |
| (14) | eine Base |
| (15) | Gemisch enthaltend ein Methioninsalz |

In der HCN-Synthese A wird im Falle des Andrussow-Verfahrens aus Methan, Ammoniak (Strom (1)) und Sauerstoff (Strom (2)) in einer exothermen Umsetzung Blausäure (HCN) und Wasser hergestellt. Im Falle des BMA-Verfahrens wird aus Methan und Ammoniak (Strom (1)) in einer endothermen Umsetzung Blausäure (HCN) und Wasserstoff hergestellt.

Das HCN-Rohgasgemisch aus der HCN-Synthese A (3) wird mit MMP (4), bevorzugt in flüssiger Form, im Schritt B in Kontakt gebracht. Nach Abtrennung des gasförmigen Teils des MMP-CN-Produktgemisches, des Abgases (6), wird der flüssige Teil des Produktgemisches (5) optional im Schritt C zwischengelagert. Das Produktgemisch enthaltend MMP-CN aus dem Schritt B wird nach einer optionalen Zwischenlagerung im Schritt C als Strom (7) in die Hydantoin-Synthese D überführt und dort mit Ammoniak und $CO_2$ oder deren Salzen zum Hydantoin umgesetzt. Dabei werden Ammoniak und $CO_2$ bevorzugt als Strom (10) aus dem nachfolgenden Schritt E eingeleitet. Das Gemisch enthaltend Hydantoin aus dem Schritt D wird über Strom (8) in den Schritt E überführt, wo das Hydantoin unter Wirkung einer Base zu einem entsprechenden Methioninsalz umgesetzt wird. Dabei wird dem Schritt E über Strom (14) eine entsprechende Base zugeführt, das Methioninsalz über den Strom (15) aus dem Schritt E entnommen. Das durch die Spaltung von Hydantoin freigesetzte Ammoniak und Kohlenstoffdioxid wird über Strom (9) dem Schritt E entnommen. Ein Teil dieses Gemisches kann optional als Strom (13) zur Entsorgung weitergeleitet, ein weiterer Teil kann in den Schritt D zurückgeführt und noch ein Teil kann als Strom (11) zu einem optionalen Aufreinigungsschritt F überführt werden. Der im Schritt F in geeignetem Maße aufgereinigte Ammoniak kann in den Schritt A zurückgeführt oder anderweitig eingesetzt werden.

Der erfindungsgemäße Schritt B, in dem ein Gasgemisch enthaltend HCN und Ammoniak mit MMP in Kontakt gebracht wird und dadurch ein Produktgemisch umfassend MMP-CN entsteht, kann auf unterschiedliche Weise gestaltet

werden.

Eine mögliche Ausführungsform des Schritts B ist eine Absorption des gasförmigen Gemisches enthaltend HCN und Ammoniak durch flüssiges MMP. Das In-Kontakt-Bringen eines Gasgemisches enthaltend HCN und Ammoniak mit flüssigem MMP kann in einem Absorptionsturm oder einem anderen oder mehreren dafür geeigneten Apparaten durchgeführt werden, in denen eine effiziente Durchmischung von gasförmigen und flüssigen Bestandteilen möglich ist, bei der eine relativ schnelle Umsetzung von MMP und HCN zu MMP-CN erreicht werden kann. Weitere mögliche Ausführungsformen des Schritts B beinhalten einen Rührreaktor, einen Schlaufenreaktor oder eine in Reihe geschaltete Kaskade von solchen Reaktoren. Außerdem können für die Durchführung des Schritts B neben weiteren in der Fachwelt allgemein bekannten Apparaten auch die folgenden eingesetzt werden: eine Bodenkolonne, eine Füllkörperkolonne, eine Tropfenkolonne oder ein Blasensäulenreaktor.

Das HCN und Ammoniak enthaltende Gasgemisch und das flüssige MMP werden bevorzugt im Gegenstrom zueinander in Kontakt gebracht. Wird ein Absorptionsturm für die Durchführung des Schritts B ausgewählt, dann wird das HCN und Ammoniak enthaltende Gasgemisch bevorzugt in den unteren Teil eines solchen Absorptionsturms eingeleitet, während das flüssige MMP im oberen Teil dieses Turms eingeleitet wird.

In dem Schritt B vorausgehenden Schritt A kann Cyanwasserstoff nach dem Andrussow- oder nach dem BMA-Verfahren hergestellt werden.

Beim oxidierenden Andrussow-Verfahren wird ein Gasgemisch enthaltend im Wesentlichen HCN, Ammoniak und Wasser in einer exothermen Umsetzung aus Methan, Ammoniak und Sauerstoff hergestellt. Als Sauerstoffquelle wird normalerweise Luft verwendet. Das Produktgemisch nach der HCN-Synthese enthält üblicherweise zusätzlich andere Gase wie Stickstoff, Argon, Wasserstoff, Kohlenmonoxid, Kohlendioxid und Methan.

Bei dem BMA-Verfahren wird das Gasgemisch, enthaltend im Wesentlichen HCN, Ammoniak und Wasserstoff aus Methan und Ammoniak hergestellt. Das Produktgemisch nach der HCN-Synthese kann außerdem unter anderem geringe Mengen an Stickstoff und Methan enthalten. Die beiden oben beschriebenen HCN-Synthesen werden üblicherweise an Platin-basierten Katalysatoren durchgeführt.

Der wesentliche Teil der vorliegenden Erfindung ist der Einsatz eines nicht oder nur teilweise vom Ammoniak befreiten, gasförmigen Cyanwasserstoffs direkt nach dessen Herstellung. Das HCN-Rohgasgemisch wird dabei nicht wie üblich einer sauren Wäsche unterzogen, sondern nach einer optionalen Abkühlung direkt im Schritt B gemäß der vorliegenden Erfindung zu einem Gemisch enthaltend MMP-CN umgesetzt.

Das molare Verhältnis von Ammoniak zu HCN in dem HCN und Ammoniak enthaltenden Gasgemisch, das im Schritt B umgesetzt wird, kann von 0,05 bis 1,00, vorzugsweise von 0,06 bis 0,99, bevorzugt von 0,07 bis 0,80, besonders bevorzugt von 0,08 bis 0,75, ganz besonders bevorzugt von 0,10 bis 0,70 betragen. Dieses molare Verhältnis kann dabei aus der Gaszusammensetzung des HCN und Ammoniak enthaltenden Gasgemisches ermittelt werden:

$$NH_3/HCN\ (mol/mol) =$$

$$[NH_3\ (mol\%)\ im\ Gemisch]/[HCN\ (mol\%)\ im\ Gemisch] =$$

$$[NH_3\ (Vol\%)\ im\ Gemisch]/[HCN\ (Vol\%)\ im\ Gemisch] =$$

$$[NH_3\ (Gew\%)\ im\ Gemisch \times M(HCN)]/[HCN\ (Gew\%)\ im\ Gemisch \times M(NH_3)],$$

wobei M(HCN) = 27,025 g/mol und $M(NH_3)$ = 17,031 g/mol die Molmassen von HCN und Ammoniak sind.

Das HCN-Rohgasgemisch liegt nach der Herstellung nach Andrussow oder BMA bei Temperaturen von typischerweise >1000 °C vor und wird daher sofort abgekühlt, um eine Zersetzung des Cyanwasserstoffs zu vermeiden. Diese vor dem Schritt B stattfindende Abkühlung des HCN-haltigen Gasgemisches kann beispielsweise mittels eines oder mehrerer in Reihe geschalteter Wärmetauscher erfolgen. Dabei kann die Energie dem HCN-Rohgasgemisch stufenweise oder kontinuierlich entnommen und optional an anderer Stelle im Prozess oder extern wiederverwendet werden. Die Temperatur des HCN-haltigen Gasstroms unmittelbar vor dem Schritt B kann von 0 bis 800 °C, bevorzugt von 10 bis 500 °C, besonders bevorzugt von 20 bis 300 °C betragen. Die bei der Durchführung des Schritts B eingehaltene Temperatur kann von 0 bis 300 °C, bevorzugt von 0 bis 100 °C, besonders bevorzugt von 0 bis 80 °C betragen.

Das nach dem Schritt B gewonnene Produktgemisch enthaltend MMP-CN kann im und/oder nach dem Schritt B in ein gasförmiges und ein flüssiges Gemisch getrennt werden. Das gasförmige Gemisch (Abgas), umfassend Stickstoff, Wasserstoff und/oder Methan, wird optional weiter aufgereinigt. Es kann als ein Brenngas für Energiegewinnung oder für die Gewinnung von einzelnen Bestandteilen, zum Beispiel Wasserstoff, verwendet werden. Das flüssige Gemisch, umfassend MMP-CN, kann wie unten beschrieben in nachfolgenden Schritten genutzt werden.

Überraschenderweise wurde gefunden, dass keine Polymerisation von HCN stattfindet, wenn das HCN-Rohgasgemisch direkt mit MMP in Kontakt gebracht wird statt wie üblich sauer gewaschen zu werden. Stattdessen wird MMP schnell und nahezu vollständig zu einem Gemisch enthaltend MMP-CN und den entsprechenden Aminoderivaten 2-Amino-4-(methylthio)buttersäurenitril (MMP-AN) und 2,2'-Bis-(2-(methylmercaptoethyl)iminodiacetonitril (Iminodinitril)

als Hauptnebenprodukte umgesetzt:

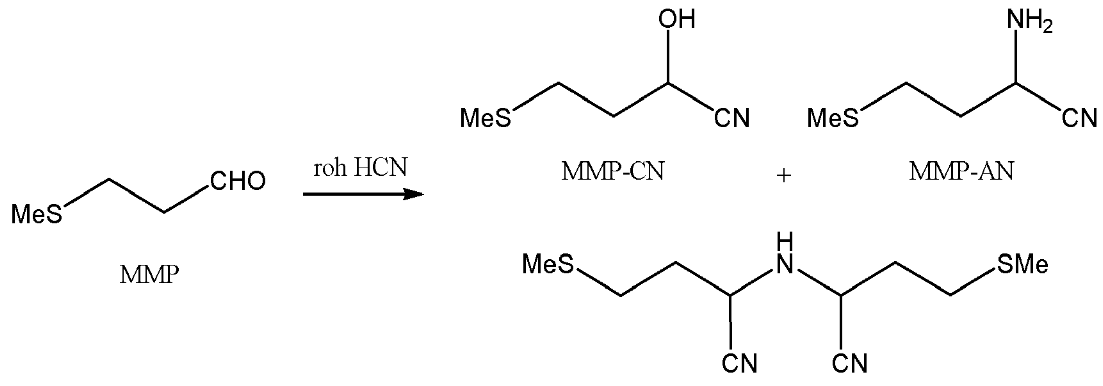


MMP

MMP-CN + MMP-AN

Iminodinitril

Bei dem erfindungsgemäßen Verfahren umfasst also das in Schritt B erhaltene Produktgemisch außer MMP-CN ferner 2-Amino-4-(methylthio)buttersäurenitril (MMP-AN) und/oder 2,2'-Bis-(2-(methylmercaptoethyl)iminodiacetonitril (Iminodinitril).

Das Produktgemisch aus dem Schritt B kann optional destillativ oder auf eine andere Weise, zum Beispiel durch Phasentrennung, Strippung oder Membrantechnologie, aufgereinigt werden, indem mindestens ein Teil des darin enthaltenen Wassers abgetrennt wird. Dieser Schritt ist besonders bei einer HCN-Herstellung nach dem Andrussow-Verfahren sinnvoll und kann direkt nach dem Schritt B, während der Zwischenlagerung im Schritt C oder in den danach folgenden Prozessschritten erfolgen. Somit können die nach einem Schritt A nach Andrussow-Verfahren mit folgendem Schritt B ursprünglich zweiphasigen wässrig-organischen Gemische wenn nötig einphasig (organisch) gemacht und auf einfachere Weise weiterverarbeitet werden. Wird Schritt A nach dem BMA-Verfahren durchgeführt liegt nach Schritt B ein einphasiges Gemisch vor.

Aus logistischen und verfahrenstechnischen Gründen kann es vorteilhaft oder notwendig sein, ein Gemisch enthaltend MMP-CN nicht direkt nach dessen Herstellung, sondern zeitversetzt, nach einer Zwischenlagerung C in weiteren Prozessschritten der Methioninsynthese umzusetzen. Dabei ist es wichtig, dass das Zwischenprodukt MMP-CN über relativ lange Zeit stabil, das heißt ohne eine beträchtliche Zersetzung von Cyanhydrin lagerfähig bleibt. Das erfindungsgemäße Verfahren kann daher ferner einen Schritt C umfassen, bei dem das in Schritt B erhaltene Produktgemisch bei Temperaturen nicht höher als 60 °C, bevorzugt nicht höher als 40 °C, besonders bevorzugt nicht höher als 20 °C und einem pH Wert von 2 bis 8, bevorzugt von 4 bis 7 gelagert wird, bevor dieses Produktgemisch weiter umgesetzt wird.

Der pH Wert des Produktgemisches nach dem Schritt B ergibt sich vor allem aus dem im Schritt B verwendeten molaren Verhältnis von Ammoniak zu Cyanwasserstoff und kann abhängig vom genutzten HCN-Verfahren (Andrussow oder BMA) oder Prozessparametern variieren.

Der pH Wert des Produktgemisches kann sich außerdem während der Schritte B und C ändern. So kann der pH Wert zum Beispiel sinken, solange die Umsetzung von Ammoniak zu MMP-AN und Iminodinitril fortschreitet. Benutzt man einen Absorptionsturm für die Durchführung des Schrittes B, so kann dadurch ein pH-Gradient entlang des Absorptionsturms entstehen.

Das Produktgemisch, enthaltend MMP-CN, kann nach dem Schritt B und einem optionalen Schritt C in einem Schritt D mit Ammoniak und/oder Ammoniumsalzen und Kohlenstoffdioxid und/oder Kohlensäuresalzen zu 5-(2-Methylmercaptoethyl)-hydantoin (Hydantoin) umgesetzt werden. Diese Umsetzung zum Hydantoin kann zum Beispiel in einem gerührten Druckreaktor bei erhöhter Temperatur stattfinden. Das für diese Umsetzung erforderliche Ammoniak und Kohlenstoffdioxid kann in den Schritt D als entsprechende Gase einzeln oder als Gemisch, optional mit Wasser, oder zumindest teilweise als die entsprechenden Salze, Ammoniumkarbonat und/oder Ammoniumhydrogenkarbonat oder dessen wässrige Lösungen eingeführt werden. In wässrigen Lösungen stehen Ammoniak, Kohlenstoffdioxid, Karbonat-, Hydrogenkarbonat- und Ammoniumionen im Gleichgewicht miteinander. Zur Vereinfachung wird in der weiteren Beschreibung nur noch Ammoniak und $CO_2$ genannt.

Es konnte gezeigt werden, dass nicht nur MMP-CN, sondern auch die oben genannten Hauptnebenprodukte MMP-AN und im Besonderen überraschender Weise auch das relativ stabile Iminodinitril erfolgreich zum Hydantoin umgesetzt werden können.

Im Schritt E kann das Hydantoin basisch unter Bildung von Kohlenstoffdioxid und Ammoniak zu mindestens einem Methioninsalz umgesetzt werden. Als Base können zum Beispiel Oxide, Hydroxide, Karbonate oder andere Salze

der Alkali-, Erdalkalimetalle, der seltenen Erden oder Ammoniumsalze sowie Gemische dieser Basen eingesetzt werden. Besonders bevorzugt werden Karbonate oder Hydrogenkarbonate der Alkalimetalle oder Ammonium eingesetzt, davon besonders bevorzugt Kalium-, Natrium- oder Ammoniumkarbonat oder deren Gemische.

Der Weg des im erfindungsgemäßen Verfahren eingesetzten und teilweise wiederverwendeten Ammoniaks spielt eine besondere Rolle. Der Ammoniak und das Kohlenstoffdioxid aus dem erfindungsgemäßen Schritt E kann zumindest teilweise in den Schritt D zurückgeführt werden. Bevorzugt wird im Schritt D nur das aus dem Schritt E zurückgeführte Ammoniak und Kohlenstoffdioxid eingesetzt.

Der Ammoniak aus dem erfindungsgemäßen Schritt E kann zumindest teilweise in den Schritt A zurückgeführt werden. Dort kann der so zurückgeführte Ammoniak wieder für die HCN-Herstellung verwendet werden. Bevorzugt wird in diesem Fall der Ammoniak von schwefelhaltigen Verbindungen und optional von $CO_2$ und Wasser befreit, bevor er in den Schritt A zurückgeführt wird.

Der Ammoniak aus dem erfindungsgemäßen Schritt E kann zumindest teilweise entsorgt werden. Bevorzugt wird er dabei katalytisch oder nicht katalytisch verbrannt.

Das im Schritt E gewonnene Methioninsalz kann in einem weiteren Prozessschritt mit einer Säure zu Methionin umgesetzt werden. Das erfindungsgemäße Verfahren zur Herstellung von Methionin oder einem Salz des Methionins kann diskontinuierlich oder kontinuierlich, bevorzugt kontinuierlich durchgeführt werden. Die kontinuierliche Umsetzung von Hydantoin über ein Methioninsalz zum Methionin ist in der Literatur bekannt.

Neben der Bildung von Methionin oder Methioninsalzen kann das nach dem erfindungsgemäßen Verfahren nach den Schritten A und B erhaltene Produktgemisch umfassend MMP-CN auch in weiteren Prozessschritten zum Beispiel nach US 20110295006 A1 zum 3,6-Bis (2'-methylmercaptoethyl)-2,5-diketopiperazin (DKP) oder nach DE 2261926 A1 zum Dipeptid von Methionin, Methionyl-Methionin (Met-Met) umgesetzt werden.

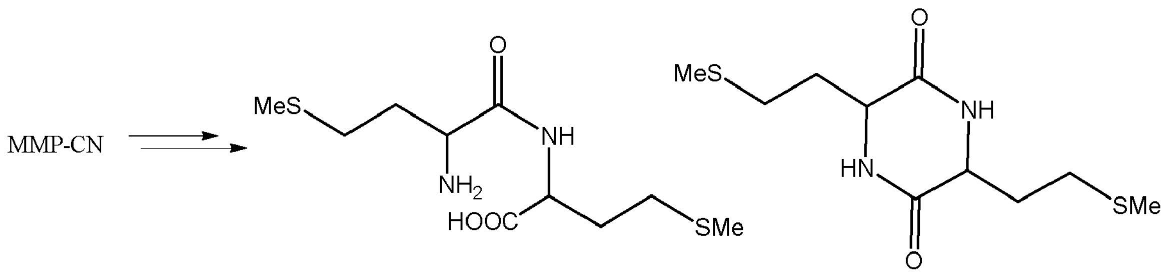


Met-Met DKP

## Beispiele

### Verwendete Methoden

#### Bestimmung von freiem Ammoniak mittels Neutralisationstitration

Der Gehalt an physikalisch gelöstem Ammoniak in MMP-CN wurde mittels Neutralisationstitration mit Salzsäure mit potentiometrischer Indikation des Äquivalenzpunkts ermittelt.

Hierzu wurden ca. 800 mg der Probe als Lösung in destilliertem Wasser (5-10 mL) vorgelegt (Kunststoffeinwegspritze) und mit 0,1 molarer Salzsäure titriert. Der Äquivalenzpunkt wurde mittels pH-Elektrode ermittelt. Die Durchführung der Neutralisationstitration ist dem Fachmann bekannt.

#### Bestimmung von freier Blausäure mittels Titration

Der Gehalt an freier Blausäure in MMP-CN wurde mittels argentometrischer Rücktitration mit Ammoniumthiocyanat unter potentiometrischer Indikation des Endpunktes (Volhard-Titration) ermittelt.

Hierzu wurden im Titriergefäß 70 mL 2-Propanol/Eisessig-Mischung (5,715 mL Eisessig in 2,5 L 2-Propanol), 10 mL 0,1 molare Silbernitrat-Lösung und ca. 4 g Probe vorgelegt, mit 1 mL Eisessig versetzt und mit 0,1 molarer Ammoniumthiocyanat-Maßlösung bis zum Endpunkt titriert. Die Bestimmung der genauen Probeneinwaage erfolgte durch Differenzwägung. Die Durchführung der Volhard-Titration ist dem Fachmann bekannt.

#### Bestimmung des Wassergehalts mittels Karl-Fischer-Titration

Der Gehalt an $H_2O$ in MMP-CN wurde nach der Methode der Titration mit biamperometrischer Indikation des Endpunktes (Karl-Fischer-Titration) bestimmt.

Hierzu wurden im Titriergefäß 20-30 mL Titriermedium (z.B. Hydranal Solvent 5, Fa. Fluka) vorgelegt und mit Titriermittel (z.B. Hydranal Titrant 5, Fa. Fluka) trockentitriert. Eine Probenmenge von ca. 500 mg wurde der austitrierten Vorlage zugesetzt (Kunststoffeinwegspritze) und mit Titriermittel bis zum Endpunkt titriert. Die Bestimmung der genauen Probeneinwaage erfolgte durch Differenzwägung.

Die Durchführung dieser Standardmethode ist dem Fachmann bekannt (siehe z.B. P. A. Bruttel, R. Schlink: *Wasserbestimmung durch Karl-Fischer-Titration* Metrohm AG).

**High Performance Liquid Chromatography (HPLC)**

Der Großteil der chromatographischen Untersuchungen (MMP-Cyanhydrin, MMP, MMP-Aminonitril, Methionin, Methioninamid, Hydantoin, Hydantoinamid, Met-Met, Methionindiketopiperazin) wurden mittels HPLC der Firma JASCO an einer RP-18 Säule (250 x 4,6 mm; 5 μm) mit anschließender UV Detektion bei 210 nm durchgeführt. Als Laufmittel diente ein phosphorsaures Acetonitril-Wasser-Gemisch (3,3 g $H_3PO_4$, 6,8 g Acetonitril, 89,9 g $H_2O$). Bei einem Fluss von 1 mL/min wurden 10 μL der jeweiligen Probelösung (50 mg Probe in 25 mL $H_2O$) injiziert. Die Kalibrierung erfolgte im Vorfeld durch die Injektion geeigneter Kalibrierlösungen, die Auswertung erfolgte durch Peakflächenvergleich mittels der externen Standardmethode. Die Durchführung der Standardmethode ist dem Fachmann bekannt.

Die Bestimmung von Iminodinitril erfolgte an oben genanntem HPLC-System mit identischer Säule, Fluss und Detektion. Als Laufmittel diente in diesem Fall ein Gemisch aus Methanol und Wasser (jeweils 50 Gew%). Es wurden 10 OL der jeweiligen Probelösung (250 mg Probe in 25 mL Laufmittel) injiziert.

**Beispiel 1**

**Herstellung von MMP-Cyanhydrin aus ammoniakhaltiger Blausäure**

188 g 3-Methylthiopropionaldehyd (Methylmercaptopropionaldehyd, MMP) (94,0 Gew%, 1,00 Äquiv.) aus industrieller Herstellung wurden mit einer Geschwindigkeit von 6 g/min auf den Kopf einer auf 55 °C temperierten Glockenbodenkolonne (5 Böden, Doppelmantel) mit Rückflusskühler dosiert. Am Boden der Kolonne wurde im Gegenstromprinzip ein Gasgemisch bestehend aus Blausäure (47,0 g, 1,02 Äquiv. bezogen auf MMP, 90 g/h), Ammoniak (7,99 g, 0,28 Äquiv. bezogen auf MMP, 15,3 g/h), Wasserdampf (80 g, 156 g/h) und Stickstoff (230 NL, 450 NL/h) eingeleitet ($NH_3$:HCN= 0,27 mol/mol). Das Produkt wurde in einem am Boden der Kolonne befestigtem Kolben gesammelt und nach Beendigung der MMP-Dosierung (ca. 30 min) analysiert. Es wurde ein klares, farbloses zweiphasiges Produkt enthalten. HPLC Analyse ergab einen Gesamtgehalt von 145 g MMP-Cyanhydrin (MMP-CN, 65,1 % bezogen auf eingesetztes MMP), 33,8 g MMP-Aminonitril (MMP-AN, 15,3 % bezogen auf eingesetztes MMP) und 30,6 g Iminodinitril (14,8 % bezogen auf eingesetztes MMP). MMP-CN, MMP-AN und Iminodinitril werden weiterhin als MMP-CN Äquivalente bezeichnet.

**Vergleichsbeispiel 1**

188 g 3-Methylthiopropionaldehyd (Methylmercaptopropionaldehyd, MMP) (94,0 Gew%, 1,00 Äquiv.) aus industrieller Herstellung wurden mit einer Geschwindigkeit von 6 g/min auf den Kopf einer auf 55 °C temperierten Glockenbodenkolonne (5 Böden, Doppelmantel) mit Rückflusskühler dosiert. Am Boden der Kolonne wurde im Gegenstromprinzip Blausäure (47,0 g, 1,02 Äquiv. bezogen auf MMP, 90 g/h) und Stickstoff (230 NL, 450 NL/h) eingeleitet. Das Produkt wurde in einem am Boden der Kolonne befestigtem Kolben gesammelt und nach Beendigung der MMP-Dosierung (ca. 30 min) analysiert. HPLC Analyse des klaren, farblosen Reaktionsproduktes (224,0 g) ergab einen MMP-Cyanhydrin-Gehalt von 95,4 Gew% (96,1 % bezogen auf eingesetztes MMP).

**Beispiel 2**

**Stabilitätsstudien von MMP-Cyanhydrin aus ammoniakhaltiger Blausäure**

In einem mit einem Eisbad gekühlten Dreihalskolben, ausgestattet mit Intensivkühler und Thermometer, wurden 79,0 g 3-Methylthiopropionaldehyd (96,3 Gew%, 1,0 Äquiv.) aus industrieller Herstellung magnetisch gerührt. Ein aus 20,8 g Blausäure (1,05 Äquiv.) und 2,12 g Ammoniak (0,17 Äquiv.) bestehendes Gasgemisch wurde eingeleitet ($NH_3$:HCN= 0,16 mol/mol), die Zugabegeschwindigkeit dabei so reguliert, dass die Temperatur im Reaktionsgefäß nie 40 °C überstieg. Nach beendeter Zugabe wurde 15 min bei Raumtemperatur gerührt. Die erhaltene farblose Substanz wurde auf drei Schottflaschen aufgeteilt und bei 10 °C, Raumtemperatur bzw. 60 °C für 10 Wochen gelagert. Die Ergebnisse der Analytik nach 1 Tag, 1 Woche und 10 Wochen sind in der nachfolgenden Tabelle 4 zusammengefasst.

Tabelle 4:

| Lagertemperatur | Lagerzeit | HPLC [Gew%] | | | Titration [Gew%] | | |
|---|---|---|---|---|---|---|---|
| | | MMP-CN | MMP-AN | Dinitril | $H_2O$ | $NH_3$ | HCN |
| 10 °C | 1 Tag | 67,3 | 7,6 | 13,3 | 5,61 | 0,01 | 0,82 |
| | 1 Woche | 59,7 | 2,0 | 23,6 | 6,21 | 0,01 | 0,77 |
| | 10 Wochen | 60,3 | 0,8 | 25,9 | 6,66 | 0,06 | 1,21 |
| RT | 1 Tag | 59,5 | 2,2 | 23,0 | 6,18 | 0,02 | 0,75 |
| | 1 Woche | 58,0 | 1,1 | 25,6 | 6,17 | 0,01 | 1,12 |
| | 10 Wochen | 52,9 | 0,9 | 28,7 | 6,36 | 0,08 | 1,42 |
| 60 °C | 1 Tag | 58,6 | 1,0 | 26,4 | 5,48 | 0,03 | 0,72 |
| | 1 Woche | 53,8 | 0,7 | 27,3 | 4,97 | 0,01 | 1,22 |
| | 10 Wochen | 52,1 | 0,0 | 28,5 | 4,99 | 0,08 | 1,56 |

Die in Tabelle 4 zusammengefassten Ergebnisse aus Beispiel 2 zeigen, dass erfindungsgemäß erhaltenes MMP-CN haltiges Produktgemisch über eine längere Zeit (mindestens bis 10 Wochen) bei Temperaturen bis 60 °C haltbar und somit für die nachfolgenden Methionin-Herstellungsschritte einsetzbar ist.

**Beispiel 3**

**Herstellung von Hydantoin aus mit ammoniakalischer Blausäure hergestelltem MMP-Cyanhydrin**

35,0 g eines mit ammoniakalischer Blausäure ($NH_3$:HCN= 0,17 mol/mol) hergestellten MMP-Cyanhydrins, bestehend aus 60,1 Gew% MMP-CN, 3,7 Gew% MMP-AN und 20,9 Gew% Iminodinitril, wurde in einem mit einem Rührfisch bestückten 300 mL Autoklavbecher mit destilliertem Wasser (52,0 g), Ammoniumcarbonat (11,9 g) und Ammoniumhydrogencarbonat (30,2 g) versetzt. Das Reaktionsgefäß wurde in einen Hochdruck-Laborautoklaven der Firma ROTH, bestückt mit Manometer, Heizung, Temperaturfühler und Druckablass, überführt. Der Autoklav wurde fest verschlossen, unter Rühren innerhalb von 15 min auf 105 °C aufgeheizt und dann weitere 20 min auf dieser Temperatur gehalten. Nach Ablauf der Reaktionszeit wurde der Autoklav unter fließendem Wasser auf Raumtemperatur abgekühlt und der entstandene Druck (ca. 17 bar) abgelassen. HPLC-Analyse des Reaktionsproduktes (121,3 g) ergab einen Gehalt von 24,8 Gew% Hydantoin (74,9% Ausbeute bezogen auf eingesetzte MMP-CN Äquivalente) und 6,5 Gew% Hydantoinamid (17,9% Ausbeute bezogen auf eingesetzte MMP-CN Äquivalente).

**Vergleichsbeispiel 3**

Ein Vergleichsexperiment zur Herstellung von Hydantoin analog dem Beispiel 3 aber mit aus ammoniakfreier Blausäure hergestelltem MMP-Cyanhydrin (35,0 g, 88 Gew%) ergab bei 104,7 g Reaktionsprodukt eine Zusammensetzung von 27,9 Gew% Hydantoin (71,4% Ausbeute bezogen auf eingesetztes MMP-CN) und 8,2 Gew% Hydantoinamid (19,1% Ausbeute bezogen auf eingesetztes MMP-CN).

**Beispiel 4**

**Herstellung von Methionin aus mit ammoniakalischer Blausäure hergestelltem MMP-Cyanhydrin**

35,0 g eines mit ammoniakalischer Blausäure ($NH_3$:HCN= 0,17 mol/mol) hergestelltem MMP-Cyanhydrins, bestehend aus 64,5 Gew% MMP-CN, 4,6 Gew% MMP-AN und 19,7 Gew% Iminodinitril, wurde in einem mit einem Rührfisch bestückten 300 mL Autoklavbecher mit destilliertem Wasser (39,0 g), Ammoniumcarbonat (13,9 g) und Ammoniumhydrogencarbonat (23,4 g) versetzt. Das Reaktionsgefäß wurde in einen Hochdruck-Laborautoklaven der Firma ROTH, bestückt mit Manometer, Heizung, Temperaturfühler, Einleitrohr und Druckablass, überführt. Der Autoklav wurde fest verschlossen, unter Rühren innerhalb von 15 min auf 105 °C aufgeheizt und dann weitere 20 min auf dieser Temperatur gehalten. Nach Ablauf der Reaktionszeit wurde der Autoklav im Wasserbad auf 70 °C abgekühlt und der entstandene Druck (ca. 15 bar) abgelassen. Nun wurde über das Einleitrohr 40 g wässrige KOH-Lösung (15 g KOH in 25

g $H_2O$) über einen Zeitraum von 10 min zudosiert. Nach beendigter Zugabe wurde der Autoklav unter Rühren innerhalb von 25 min auf 180 °C geheizt und dann weitere 30 min auf dieser Temperatur gehalten. Während der Reaktionszeit wurde der Druck ca. alle 5 min, mindestens aber bei Überschreitung von 10 bar, auf 5 bar abgelassen. Nach Ablauf der Reaktionszeit wurde der Autoklav unter fließendem Wasser auf Raumtemperatur abgekühlt und auf Normdruck entspannt. HPLC-Analyse des Reaktionsproduktes (118,8 g) ergab einen Gehalt von 16,6 Gew% Methionin (54,8% Ausbeute bezogen auf eingesetzte MMP-Äquivalente), 0,7 Gew% Methioninamid (2,3% Ausbeute bezogen auf eingesetzte MMP-Äquivalente), 7,1 Gew% Methionylmethionin (24,9% Ausbeute bezogen auf eingesetzte MMP-CN Äquivalente) und 0,4 Gew% Methionindiketopiperazin (1,6% Ausbeute bezogen auf eingesetzte MMP-CN Äquivalente).

**Vergleichsbeispiel 4**

Ein Vergleichsexperiment mit aus ammoniakfreier Blausäure hergestelltem MMP-Cyanhydrin (35,0 g, 95,4 Gew%) ergab bei 142,2 g Reaktionsprodukt eine Zusammensetzung von 15,1 Gew% Methionin (56,5% Ausbeute bezogen auf eingesetzte MMP-Äquivalente), 1,1 Gew% Methioninamid (4,1% Ausbeute bezogen auf eingesetzte MMP-CN Äquivalente), 6,2 Gew% Methionylmethionin (24,7% Ausbeute bezogen auf eingesetzte MMP-CN Äquivalente) und 0,6 Gew% Methionindiketopiperazin (2,6% Ausbeute bezogen auf eingesetzte MMP-CN Äquivalente).

Tabelle 5. Gegenüberstellung der MMP-CN-, Hydantoin- und Methionin-Herstellung aus ammoniakfreier vs. ammoniakhaltiger Blausäure:

| | Ausbeute, % | | |
|---|---|---|---|
| | MMP-CN (MMP-AN; Iminodinitril) | Hydantoin (Hydantoinamid) | Methionin (Met-Amid; Met-Met; Diketopiperazin) |
| Beispiel 1 | 65,1(15,3; 14,8) | | |
| Vergleichsbeispiel 1 | 96,1 | | |
| Beispiel 3 | | 74,9 (17,9) | |
| Vergleichsbeispiel 3 | | 71,4 (19,1) | |
| Beispiel 4 | | | 54,8 (2,3; 24,9; 1,6) |
| Vergleichsbeispiel 4 | | | 56,5 (4,1; 24,7; 2,6) |

Die in Tabelle 5 zusammengefassten Ergebnisse zeigen, dass die Herstellung des Zwischenprodukts Hydantoin sowie des Endprodukts Methionin aus MMP-Cyanhydrin aus ammoniakhaltiger (Beispiele 3, 4) und ammoniakfreier Blausäure (Vergleichsbeispiele 3, 4) vergleichbare Ausbeuten und Nebenproduktspektren liefern.

**Patentansprüche**

1. Verfahren zur Herstellung von 2-Hydroxy-4-(methylthio)buttersäurenitril (MMP-CN), umfassend einen Schritt B, in dem ein Gasgemisch enthaltend Cyanwasserstoff (HCN) und Ammoniak mit 3-Methylmercaptopropionaldehyd (MMP) in Kontakt gebracht wird und dadurch ein Produktgemisch umfassend 2-Hydroxy-4-(methylthio)buttersäurenitril (MMP-CN) erhalten wird.

2. Verfahren zur Herstellung von Methionin oder einem Salz des Methionins, umfassend einen Schritt B, in dem ein Gasgemisch enthaltend Cyanwasserstoff (HCN) und Ammoniak mit 3-Methylmercaptopropionaldehyd (MMP) in Kontakt gebracht wird und dadurch ein Produktgemisch umfassend 2-Hydroxy-4-(methylthio)buttersäurenitril (MMP-CN) erhalten wird.

3. Verfahren nach Anspruch 1 oder 2, bei dem das molare Verhältnis von Ammoniak zu HCN im Gasgemisch von 0,06 bis 0,99 ist.

4. Verfahren nach einem der Ansprüche 1 bis 3, ferner umfassend den dem Schritt B vorausgehenden Schritt A, in dem das Gasgemisch, enthaltend im Wesentlichen HCN, Ammoniak und Wasser nach dem Andrussow-Verfahren aus Methan, Ammoniak und Sauerstoff hergestellt wird.

5. Verfahren nach einem der Ansprüche 1 bis 3, ferner umfassend den dem Schritt B vorausgehenden Schritt A, in

dem das Gasgemisch, enthaltend im Wesentlichen HCN, Ammoniak und Wasserstoff nach dem BMA-Verfahren aus Methan und Ammoniak hergestellt wird.

6. Verfahren nach einem der Ansprüche 1 bis 5, bei dem das nach dem Schritt B gewonnene Produktgemisch im und/oder nach dem Schritt B in ein Abgas, umfassend Stickstoff, Wasserstoff und/oder Methan, und ein flüssiges Gemisch, umfassend MMP-CN, getrennt wird und das Abgas optional weiter gereinigt wird.

7. Verfahren nach einem der Ansprüche 1 bis 6, bei dem das in Schritt B erhaltene Produktgemisch ferner 2-Amino-4-(methylthio)buttersäurenitril (MMP-AN) und/oder 2,2'-Bis-(2-(methylmercaptoethyl)-iminodiacetonitril (Iminodinitril) umfasst.

8. Verfahren nach einem der Ansprüche 2 bis 7 ferner umfassend einen Schritt C, bei dem das in Schritt B erhaltene Produktgemisch bei Temperaturen nicht höher als 60 °C und einem pH Wert von 2 bis 8 gelagert wird, bevor dieses Produktgemisch weiter umgesetzt wird.

9. Verfahren nach einem der Ansprüche 2 bis 7, umfassend ferner einen Schritt D, in dem das in Schritt B erhaltene Produktgemisch mit Ammoniak und/oder Ammoniumsalzen und Kohlenstoffdioxid und/oder Kohlensäuresalzen zu 5-(2-Methylmercaptoethyl)-hydantoin (Hydantoin) umgesetzt wird.

10. Verfahren nach Anspruch 9, ferner umfassend einen Schritt E in dem das Hydantoin basisch unter Bildung von Kohlenstoffdioxid und Ammoniak zu mindestens einem Methioninsalz umgesetzt wird.

11. Verfahren nach Anspruch 10, bei dem der Ammoniak und das Kohlenstoffdioxid aus Schritt E zumindest teilweise in den Schritt D zurückgeführt werden.

12. Verfahren nach Anspruch 10, bei dem der in Schritt E gewonnene Ammoniak zumindest teilweise in den Schritt A zurückgeführt wird.

13. Verfahren nach Anspruch 10, bei dem der in Schritt E gewonnene Ammoniak zumindest teilweise entsorgt wird.

14. Verfahren nach einem der Ansprüche 2 bis 13, bei dem das Produktgemisch aus dem Schritt B destillativ aufgereinigt wird, indem mindestens ein Teil des darin enthaltenen Wassers abgetrennt wird.

15. Verfahren nach Anspruch 12, bei dem der nach dem Schritt E gewonnene Ammoniak von schwefelhaltigen Verbindungen und optional von $CO_2$ befreit wird, bevor er in den Schritt A zurückgeführt wird.

16. Verfahren nach Anspruch 10, bei dem das Methioninsalz mit einer Säure zu Methionin umgesetzt wird.

17. Verwendung von nach Ansprüchen 1 oder 2 hergestelltem Methionin, Salz des Methionins und/oder MMP-CN in einem Verfahren zur Herstellung vom Dipeptid des Methionins, Methionyl-Methionin (Met-Met).

18. Verwendung von nach Ansprüchen 1 oder 2 hergestelltem Methionin, Salz des Methionins und/oder MMP-CN in einem Verfahren zur Herstellung vom 3,6-Bis (2'-methylmercaptoethyl)-2,5-diketopiperazin (DKP).

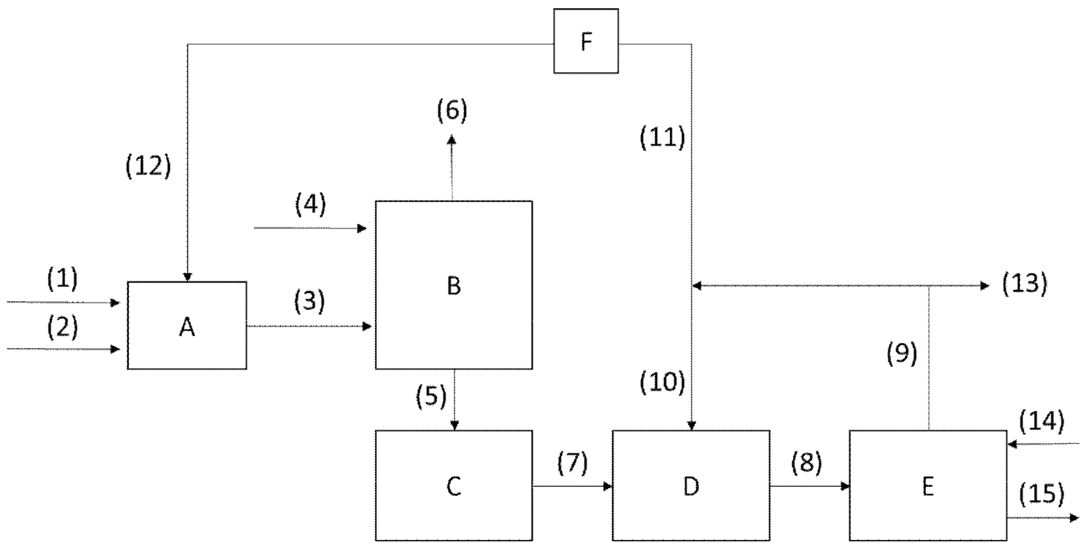


Fig.1

Europäisches Patentamt
European Patent Office
Office européen des brevets

# EUROPÄISCHER RECHERCHENBERICHT

Nummer der Anmeldung
EP 16 20 5829

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | JP 2002 105048 A (SUMITOMO CHEMICAL)<br>10. April 2002 (2002-04-10)<br>* Absatz [0015] *<br>----- | 1-16 | INV.<br>C07C319/20<br>C07C323/60<br>C01C3/02<br>C07C323/58<br>C07D233/76 |
| A | CN 102 399 177 A (KUANYI LI)<br>4. April 2012 (2012-04-04)<br>* Absatz [0055] *<br>----- | 1-16 | |
| X | WO 2010/043558 A1 (EVONIK DEGUSSA)<br>22. April 2010 (2010-04-22)<br>* Beispiel 19 *<br>----- | 17,18 | |

RECHERCHIERTE SACHGEBIETE (IPC)
C01C
C07C
C07D

Der vorliegende Recherchenbericht wurde für alle Patentansprüche erstellt

1

| Recherchenort | Abschlußdatum der Recherche | Prüfer |
|---|---|---|
| Den Haag | 24. Mai 2017 | English, Russell |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 16 20 5829

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-05-2017

| Im Recherchenbericht angeführtes Patentdokument | Datum der Veröffentlichung | Mitglied(er) der Patentfamilie | Datum der Veröffentlichung |
|---|---|---|---|
| JP 2002105048 A | 10-04-2002 | JP 4517486 B2 | 04-08-2010 |
| | | JP 2002105048 A | 10-04-2002 |
| CN 102399177 A | 04-04-2012 | KEINE | |
| WO 2010043558 A1 | 22-04-2010 | BR PI0920882 A2 | 21-02-2017 |
| | | CA 2740663 A1 | 22-04-2010 |
| | | CN 101720858 A | 09-06-2010 |
| | | CN 102186358 A | 14-09-2011 |
| | | CN 105053577 A | 18-11-2015 |
| | | CN 105061555 A | 18-11-2015 |
| | | DE 102008042932 A1 | 22-04-2010 |
| | | EP 2348881 A1 | 03-08-2011 |
| | | ES 2437602 T3 | 13-01-2014 |
| | | JP 5693457 B2 | 01-04-2015 |
| | | JP 6033254 B2 | 30-11-2016 |
| | | JP 2012505646 A | 08-03-2012 |
| | | JP 2014193176 A | 09-10-2014 |
| | | MY 155599 A | 13-11-2015 |
| | | RU 2011119472 A | 20-03-2013 |
| | | US 2010098801 A1 | 22-04-2010 |
| | | US 2013008384 A1 | 10-01-2013 |
| | | US 2013011514 A1 | 10-01-2013 |
| | | US 2015223495 A1 | 13-08-2015 |
| | | WO 2010043558 A1 | 22-04-2010 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0780370 A2
- JP 2003104959 A
- JP 2003104960 A
- GB 936664 A
- GB 1108926 A
- WO 2012113664 A1
- WO 2012113665 A1
- DE 102007034715 A1
- DE 1041476
- CN 103408498 A
- US 2590146 A
- US 2899274 A
- US 3112177 A
- US 2797148 A
- US 3914386 A
- US 5756803 A
- US 2745745 A
- CN 103420883 A
- JP 2002105048 B
- US 20110295006 A1
- DE 2261926 A1

### In der Beschreibung aufgeführte Nicht-Patentliteratur

- Ullmann's Encyclopedia of Industrial Chemistry. Amino Acids. 15. April 2007
- *Chemie-Ing.-Techn.,* 1953, vol. 12, 697-701
- *Chemie-Ing.-Techn.,* 1958, vol. 5, 305-310
- Hydrocyanic Acid (HCN) Production. Handbook of Heterogeneous Catalysis. vol. 5, 2592-2609
- Ullmann's Encyclopedia of Industrial Chemistry. Cyano Compounds, Inorganic. 15. Oktober 2011
- *Laut Chemie-Ing.-Techn.,* 1953, vol. 12, 697-701